(19) Europäisches Patentamt
European Patent Office
Office européen des brevets

(11) Veröffentlichungsnummer: **0 039 484**
**A1**

(12) # EUROPÄISCHE PATENTANMELDUNG

(21) Anmeldenummer: **81103259.8**

(22) Anmeldetag: **30.04.81**

(51) Int. Cl.³: **C 07 C 43/23**, C 07 C 43/295, C 07 C 41/18, C 07 C 43/315

(30) Priorität: **07.05.80 DE 3017393**

(43) Veröffentlichungstag der Anmeldung: **11.11.81**
**Patentblatt 81/45**

(84) Benannte Vertragsstaaten: **CH DE FR GB LI**

(71) Anmelder: **HOECHST AKTIENGESELLSCHAFT,**
**Postfach 80 03 20, D-6230 Frankfurt/Main 80 (DE)**

(72) Erfinder: **Keller, Reinhold, Dr., Wiesenweg 5, D-6232 Bad Soden am Taunus (DE)**
Erfinder: **Konz, Elmar, Dr., Brüningstrasse 9, D-6233 Kelkheim (Taunus) (DE)**

(54) **Verfahren zur Herstellung von Hydrochinonmonoäthern sowie die dabei als Zwischenprodukte gebildeten Chinolketale.**

(57) Hydrochinonmonoäther werden hergestellt durch Reduktion von p-Benzochinonmonoketalen, vorzugsweise mit komplexen Hydriden des Bors oder Aluminiums, mit einem Sulfit oder molekularem Wasserstoff in Gegenwart eines Nickel-Katalysators bis zur Aufnahme von einem Mol $H_2$/Mol p-Benzochinonmonoketal in einem inerten Lösungsmittel zu den entsprechenden Chinolketalen und Behandlung derselben mit einer Säure. Die hier als Zwischenprodukte gebildeten Chinolketale sind neue Verbindungen.

Die Hydrochinonmonoäther sind Vor-, Zwischen- und Endprodukte auf verschiedenen Sachgebieten.

EP 0 039 484 A1

HOECHST AKTIENGESELLSCHAFT

Verfahren zur Herstellung von Hydrochinonmonoäthern sowie die dabei als Zwischenprodukte gebildeten Chinolketale

Monoäther sowohl des unsubstituierten als auch des substituierten Hyrochinons sind wertvolle Vor-, Zwischen- und Endprodukte auf verschiedenen Sachgebieten wie z.B. auf dem Farbstoff-, Pharma- und Pflanzenschutzsektor. Ein als Endprodukt interessanter Monoäther eines substituierten Hydrochinons ist z.B. das als krebshemmend bekannte 2-tert.-Butyl-4-hydroxyanisol (J. Med. Chem. 1979, Vol. 22, S. 569).

Zur Herstellung der Hydrochinonmonoäther ist eine Reihe verschiedener Verfahren bekannt. Von den klassischen Verfahren seien beispielsweise die Umsetzungen der Alkalisalze des (unsubstituierten und des substituierten) Hydrochinons mit Alkylierungsmitteln wie Alkylhalogeniden, Dialkylsulfaten und Toluolsulfonsäureestern erwähnt (Houben-Weyl "Methoden der Organischen Chemie" Band 6/3, S. 49 - 84) Für diese Verfahren ist jedoch nachteilig, daß sie zwecks Unterdrückung der Bis-Verätherung einen erheblichen Hydrochinon-Überschuß erfordern, was bei der Aufarbeitung des Reaktionsgemisches einen nicht unbeträchtlichen Aufwand zur Folge hat. Weiterhin ist das im Überschuß eingesetzte Hydrochinon erst nach seiner Reinigung (z.B. durch Destillation) wieder verwendbar.

Diese Nachteile hat man bereits auf verschiedene Weise zu vermeiden versucht. Nach einem derartigen verbesserten Verfahren wird u.a. Hydrochinon mit Alkylhalogeniden im Molverhältnis von etwa 1:1 im Zweiphasensystem Wasser/mit Wasser nicht mischbares Lösungsmittel (z.B. 1,2-Dichloräthan) in Gegenwart von Orthophosphat bei erhöhtem Druck und erhöhter Temperatur monoalkyliert (JP-AS 69054 vom 27.11.1975); innerhalb weniger Stunden wird der entsprechende Hydrochinonmonoäther hierbei in hohen Ausbeuten erhalten.

Wenngleich bei dem letzgenannten Verfahren kaum Bisäther als Nebenprodukte entstehen, entspricht es doch den heutigen hohen Anforderungen an die Wirtschaftlichkeit technischer Verfahren nicht ganz; denn die Durchführung des Verfahrens erfordert technisch relativ recht aufwendige Apparaturen und verhältnismäßig hohe Energien. Außerdem ist diese Methode praktisch nur für die Herstellung niederer Alkyläther geeignet.

Es war daher wünschenswert und bestand die Aufgabe, ein verbessertes Verfahren zur Herstellung von Hydrochinonmonoäthern zu finden, welches die Vorteile des letzgenannten bekannten Verfahrens besitzt (keine wesentlichen Hydrochinon-Überschüsse, keine Bisverätherung) und darüberhinaus präparativ einfacher durchführbar ist sowie auch von einfacheren - d.h. einfacher und billiger darstellbaren - Ausgangsprodukten ausgeht.

Diese Aufgabe konnte erfindungsgemäß durch Reduktion von p-Benzochinonmonoketalen zu den entsprechenden Chinolketalen und eine Säurebehandlung derselben gelöst werden.

Erfindungsgegenstand ist somit ein Verfahren zur Herstellung von Hydrochinonmonoäthern, das dadurch gekennzeichnet ist, daß man

a) p-Benzochinonmonoketale in einem inerten Lösungsmittel zu den entsprechenden Chinolketalen reduziert und
b) diese dann - gegebenenfalls nach ihrer Isolierung - mit einer Säure behandelt. Bei der Säurebehandlung erfolgt eine 1,4-Eliminierung von Alkohol oder einem

Phenol aus den Chinolketalen, wobei sich diese zu dem aromatischen System des entsprechenden Hydrochinonmonoäthers umlagern.

Es war überraschend, daß diese Reaktion glatt und mit hohen Ausbeuten an den gewünschten Hydrochinonmonoäthern gelingt, da man bisher offenbar der Meinung war, ausgehend von p-Benzochinonmonoketalen überhaupt nicht - oder zumindest nicht auf einfache Weise - zu Hydrochinonmonoäthern gelangen zu können; denn über diesbezügliche Versuche ist bisher nichts bekannt geworden.

Als Ausgangsverbindungen für das Verfahren können im Prinzip alle möglichen p-Benzochinonmonoketale (mit allen möglichen Substituenten am Chinonkern und auch mit allen möglichen Ketalgruppen) verwendet werden. Bevorzugt ist jedoch die Verwendung der unter die nachstehende Formel I fallenden p-Benzochinonmonoketale:

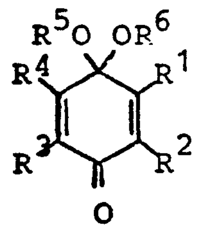

(I)

worin die Reste $R^1$ bis $R^4$ unabhängig voneinander Wasserstoff, Halogen oder-gegebenenfalls über Sauerstoff an den Chinonring gebundene-organische Reste, sowie $R^5$ und $R^6$ - ebenfalls unabhängig voneinander - gegebenenfalls durch reaktionsinerte Gruppen substituierte Alkylreste oder - zusammen - einen gegebenenfalls durch O-Atome unterbrochenen Alkylenrest oder einen Arylenrest bedeuten.

Als gegebenenfalls über Sauerstoff an den Chinonring gebundene organische Reste seien für $R^1$ bis $R^4$ in beispielhafter Weise genannt:

Alkyl, Aryl, -OAlkyl, -OAryl, CN, COOH, COOR' (R' = Alkyl oder Aryl),

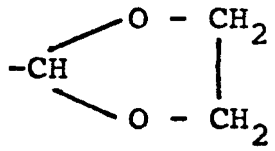 etc.

Für $R^5$ und $R^6$ kommen in Frage: unsubstituierte unverzweigte und verzweigte Alkylreste sowie substituierte Alkylreste (substituiert durch z.B. Halogen, Aryl, Alkoxy, Aryloxy, COOH etc.); wenn $R^5$ und $R^6$ zusammen einen gegebenenfalls durch O-Atome unterbrochenen Alkylenrest oder einen Arylenrest bilden, kommen folgende Reste in Frage:
Alkylenreste mit vorzugsweise 2 - 6 C-Atomen in der Kette (gegebenenfalls substituiert durch Alkyl, Alkoxy, Aryl, Aryloxy, Halogen, CN etc.),
Reste $-\underset{R''}{CH}-\underset{R''}{CH}\left[-O-\underset{R''}{CH}-\underset{R''}{CH}-\right]_n$ mit R"= H, $CH_3$ oder $C_2H_5$
und n = 0 - 3,
sowie 1,2- und 2,2'-Arylenreste wie z.B. 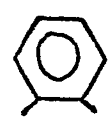 und 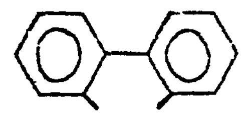 etc.

Besonders bevorzugt sind mindestens 3 der Reste $R^1$ bis $R^4$ = H und - wenn nur 3 dieser Reste = H sind - ist der 4. = Cl, Br, $(C_1-C_4)$-Alkyl oder $(C_1-C_4)$-Alkoxy; insbesondere ist der 4. Rest nur = Cl, Br oder $(C_1-C_4)$-Alkyl.

Für $R^5$ und $R^6$ ist besonders bevorzugt:
$R^5 = R^6 = (C_1-C_4)$-Alkyl sowie
$R^5 + R^6$ = Alkylengruppen mit 2 - 4 C-Atomen in der Kette oder 1,2-Phenylen.

Besonders bevorzugte Verbindungen der Formel I sind also z.B.:

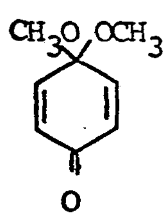 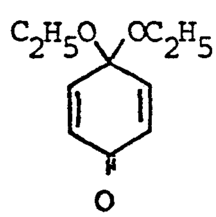 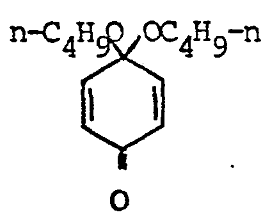 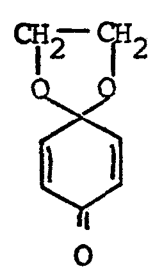

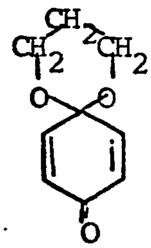 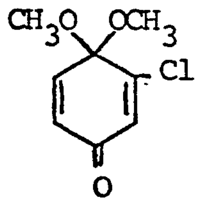 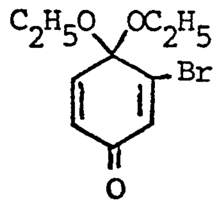 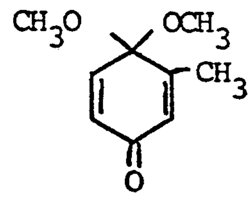

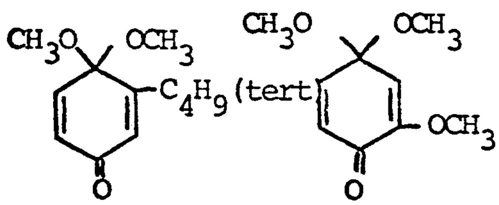 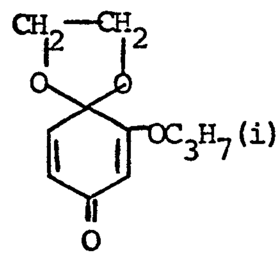 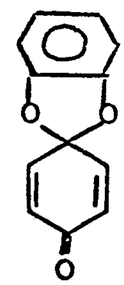 etc.

Zu den p-Benzochinonmonoketalen der Formel I kommt man im allgemeinen ausgehend von den entsprechenden p-Benzochinon-tetramethyldiketalen, die ihrerseits z.B. durch anodische Oxidation von Hydrochinondimethyläthern in methanolischer KOH an einer Platinanode gewonnen werden können (vgl. DR. Hinton B.L. Chenard und J.S. Swenton, J.C.S. Chem. Comm. 1979, 326/27). Besonders vorteilhaft werden die p-Benzochinon-tetramethyldiketale nach dem aus der DE-OS 24 60 754 bekannten - ebenfalls elektrochemischen - Verfahren hergestellt; dieses Verfahren ist allerdings dort nur für die Herstellung von unsubstituiertem sowie von Monohalogen- und Monoalkyl-substituiertem p-Benzochinon-tetramethyldiketal (aus Benzol bzw. Monohalogen- oder Monoalkyl-substituiertem Anisol) beschrieben. Wenn man von anderweitig substituiertem Benzol oder Anisol ausgeht, kann man nach diesem Verfahren aber auch solche p-Benzochinon-tetramethyldiketale erhalten, die durch andere Reste der unter die Bedeutungen von $R^1$ bis $R^4$ bei Formel I fallenden Art substituiert sind.

Aus den p-Benzochinon-tetramethyldiketalen werden die Monoketale der Formel I dann entweder durch Monoentketalisierung und gegebenenfalls (wenn Monoketale der Formel I

mit $R^5$ und $R^6$ ungleich $CH_3$ gewünscht werden) Umketalisierung oder - umgekehrt - erst durch Umketalisierung (wenn Monoketale der Formel I mit $R^5$ und $R^6 \neq CH_3$ gewünscht werden) und anschließende Monoentketalisierung hergestellt.

Die Monoentketalisierung der p-Benzochinon-tetramethyldiketale erfolgt zweckmäßig in einer wässrigen Phosphatpufferlösung vom pH etwa 5,5 bis 6. Die anderen p-Benzochinondiketale werden mit Vorteil in Aceton als Lösungsmittel in Gegenwart katalytischer Mengen einer organischen oder anorganischen Säure bei Raumtemperatur monoentketalisiert. Geeignete Monoentketalisierungs-Vorschriften finden sich z.B. in dem genannten Artikel von Hinton, Chenard und Swenton in J. C.S. Chem. Comm. 1979, 326/27 oder auch in der Arbeit von J.E. Heller et al. in Helvetica Chimica Acta Vol. 56 (1973) S. 272 bis 280.

Die Umketalisierung der p-Benzochinon-tetramethyldiketale sowie der Dimethyl-monoketale mit Alkoholen, Diolen oder Phenolen erfolgt zweckmäßig in einem inerten Lösungsmittel in Gegenwart katalytischer Mengen einer starken Säure bei Temperaturen zwischen etwa 5 und 50°C, vorzugsweise zwischen etwa 10 und 15°C.

Geeignete inerte Lösungsmittel sind hier z.B. aliphatische und cycloaliphatische Äther (Diäthyläther, Tetrahydrofuran, Dioxan etc.), Halogenkohlenwasserstoffe (Chloroform, Methylenchlorid, Tetrachlorkohlenstoff, Chlorbenzol etc.), Alkylaromaten (Toluol, Xylol etc.) u.a.

Geeignete Katalysatoren sind z.B. p-Toluolsulfonsäure und Perchlorsäure.

Eine günstige Ausführungsform der Umketalisierung besteht darin, daß man ein solches inertes Lösungsmittel verwendet, in welchem das Ausgangsketal gut, das Endketal aber weniger gut

oder möglichst überhaupt nicht löslich ist, so daß dieses dann der Gleichgewichtsreaktion auf einfache Weise entzogen wird. Diese Ausführungsform ist besonders für die Herstellung cyclischer Ketale geeignet, weil hier der Löslichkeitsunterschied zwischen Ausgangs- und Endketal relativ gut ausgeprägt ist.

In Fällen, in welchen das Endketal nicht ausfällt, muß mit einem größeren Überschuß an Alkohol bzw. Diol oder Phenol gearbeitet werden.

Die Umketalisierung dauert im allgemeinen zwischen etwa 5 und 20 Minuten.

Die p-Benzochinonmonoketale werden nach dem erfindungsgemäßen Verfahren in einem inerten Lösungsmittel zu den entsprechenden Chinolketalen reduziert.

Geeignete inerte Lösungsmittel sind hier insbesondere Wasser, $(C_1-C_3)$-Alkanole und/oder aliphatische $(C_3-C_8)$-Äther. Die Alkanole sind Methanol, Äthanol, n-und i-Propanol; beispielhafte aliphatische Äther sind Diäthyläther, Tetrahydrofuran, Dioxan, Diglyme und Triglyme.

Die Konzentration der p-Benzochinonmonoketale in den genannten inerten Lösungsmitteln ist im Prinzip nicht kritisch und je nach den Löslichkeitsverhältnissen in weiten Grenzen variierbar.

Die Reduktion wird vorzugsweise mit komplexen Hydriden des Bors oder Aluminiums ($NaBH_4$, $LiAlH_4$ etc.), mit einem Sulfit (insbesondere Ammonium- und Alkalisulfite wie z.B. $NH_4HSO_3$, $Na_2SO_3$, $K_2S_2O_5$ etc.) oder mit molekularem Wasserstoff in Gegenwart eines Nickel-Katalysators bis zur Aufnahme von einem Mol Wasserstoff/Mol p-Benzochinonmonoketal durchgeführt.

Im Falle der Reduktion mit einem komplexen Hydrid des Bors oder Aluminiums ist ein Molverhältnis von p-Benzophenonmonoketal zu komplexem Hydrid von etwa 4:1 anzuwenden, wobei das komplexe Hydrid zweckmäßig in einem geringfügigen Überschuß eingesetzt wird. Mit den komplexen Hydriden des Bors kann in Wasser oder einem Alkanol als Lösungsmittel gearbeitet werden; die Reduktion mit komplexen Hydriden des Aluminiums wird zweckmäßig in wasserfreien Äthern durchgeführt.

Für die Reduktion mit Sulfit ist mindestens ein Mol $HSO_3^-$/ Mol p-Benzochinonmonoketal einzusetzen. Ein Überschuß des Reduktionsmittels schadet auch hier nicht. Für die Reduktion mit einem Sulfit ist Wasser das geeignetste Lösungsmittel.

Bei der Reduktion mit molekularem Wasserstoff ist darauf zu achten, daß nicht mehr als 1 Mol $H_2$/Mol p-Benzochinonmonoketal angewandt wird, da andernfalls auch die Doppelbindungen des Chinonkerns hydriert werden können. Geeigneter Katalysator ist z.B. Raney-Nickel. Zweckmäßige Lösungsmittel für die katalytische Hydrierung sind Alkanole, insbesondere Methanol.

Die für die Reduktion angewandten Temperaturen liegen im allgemeinen zwischen etwa -10 und +25°C, vorzugsweise zwischen etwa +5 und +15°C. Wenn Wasser als Lösungsmittel verwendet wird, kommen jedoch praktisch nur die Temperaturen oberhalb etwa 0°C in Frage.

Bei der erfindungsgemäßen Reduktion entstehen die Chinol-ketale der Formel II

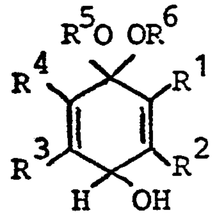

(II)

worin die Reste $R^1$ bis $R^6$ die gleiche Bedeutung wie in Formel I besitzen.

Bevorzugt sind also auch hier mindestens 3 der Reste $R^1$ bis $R^4$ = H und - wenn nur 3 dieser Reste H sind - ist der 4. bevorzugt = Cl, Br, $(C_1-C_4)$-Alkyl oder $(C_1-C_4)$-Alkoxy; insbesondere ist der 4. Rest nur Cl, Br oder $(C_1-C_4)$-Alkyl.

Bevorzugt ist weiterhin auch hier
$R^5 = R^6 = (C_1-C_4)$-Alkyl sowie
$R^5 + R^6$ = Alkylengruppen mit 2 - 4 C-Atomen in der Kette oder 1,2-Phenylen.

Diese Chinolketale sind neue Verbindungen und ebenfalls Erfindungsgegenstand.

Sie sind empfindlich gegen Säure und lagern sich in deren Gegenwart unter 1,4-Eliminierung von Alkohol bzw. Phenol schnell in das aromatische System des entsprechenden Hydrochinonmonoäthers um. Wenn der Reduktionsansatz sauer aufgearbeitet wird, wird hier gleich der gewünschte Hydrochinonmonoäther erhalten. Wenn die Reduktion etwa mit $NaBH_4$ in wässriger Lösung erfolgt, wird nach beendeter Reaktion zweckmäßig z.B. mit konzentrierter Salzsäure auf pH etwa 5 angesäuert und die Reaktionslösung im Vakuum bis zur Trockne eingedampft. Nach Aufnahme des Rückstandes mit einem organischen Lösungsmittel wie z.B. mit Äthylacetat und Abfiltration von unlöslichem Rückstand wird der Hydrochinonmonoäther aus der organischen Lösung wie üblich gewonnen.

Das erfindungsgemäße Verfahren läßt sich schematisch durch folgende Formelgleichung wiedergeben:

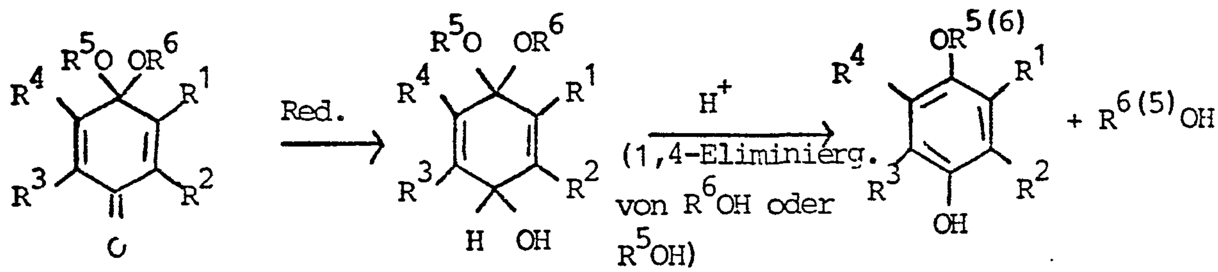

Wenn $R^5 + R^6$ zusammen einen gegebenenfalls durch O-Atome unterbrochenen Alkylenrest oder einen Arylenrest bedeuten, kann der gebildete Hydrochinonmonoäther durch folgende Formel besser wiedergegeben werden:

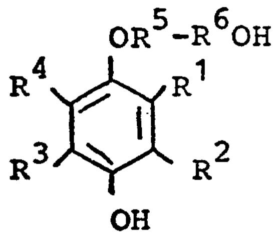

bzw.

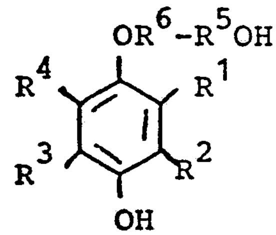

In allen Formeln besitzen $R^1$ bis $R^5$ die bei den Formeln I und II angegebenen Bedeutungen.

Wegen der einfach zugänglichen Ausgangsprodukte (insbesondere wenn diese - d.h. also die p-Benzochinonmonoketale - nach dem elektrochemischen Verfahren der DE-OS 24 60 754 hergestellt werden) sowie wegen des einfachen und hochspezifischen Verlaufs des erfindungsgemäßen Verfahrens und der hohen bis sehr hohen Ausbeuten an den gewünschten Hydrochinonmonoäthern, stellt das Verfahren einen erheblichen Fortschritt dar.

Die Erfindung wird nun durch die folgenden Beispiele näher erläutert.

Beispiel 1

a) Herstellung des Ausgangsprodukts (p-Benzochinon-monoäthylenketal)

100 g (0.5 Mol) 3,3,6,6-Tetramethoxy-1,4-cyclohexadien und 93 g (1.5 Mol) Äthylenglykol werden in 200 ml Diäthyläther aufgenommen und unter kräftigem Rühren bei 5°C mit 50 mg p-Toluolsulfonsäure versetzt. Momentan bildet sich ein weißer Niederschlag. Man rührt noch 5 Minuten, filtriert ab und wäscht mit wenig Äther nach. Ausbeute: 86 g (97 %) farblose Kristalle, Schmp. 237°C.

$C_{10}H_{12}O_4$ (196.2) ber.: C 61.22 H 6.2
gef.: C 61.1 H 6.3

IR (KBr): 2995, 2960, 2895 (CH); 1199, 1120, 1077 (C-O) $cm^{-1}$

NMR ($CDCl_3$, 60 MHz): $\tau$ = 4.05 (s, 2(3,5,6)-H); 6.00 (s, $-OCH_2CH_2O$);

80 g des so hergestellten p-Benzochinon-bis-äthylenketals werden in 300 ml Aceton aufgenommen und unter Rühren mit 2 Tropfen 70 %iger Perchlorsäure versetzt. Nach 5 Minuten ist die Lösung homogen. Man rührt noch weitere 5 Minuten, verdünnt mit 200 ml Methylenchlorid und schüttelt mit 100 ml Bicarbonat-Lösung aus. Nach Trocknen der organischen Phase über $MgSO_4$ zieht man das Lösungsmittel ab und kristallisiert den Rückstand aus Diäthyläther/Hexan (4:1) um. Ausbeute: 56 g (91 %) farblose Kristalle, Schmp. 52°C.

p-Benzochinon-monoäthylenketal
$C_8H_8O_3$ (152.1) ber.: C 63.1 G 5.3
gef.: C 63.0 H 5.3

IR ($CCl_4$): 3050, 2980, 2950, 1690, 1675, 1640 $cm^{-1}$

NMR ($CDCl_3$, 60 MHz): $\tau$ = 3.56 (AB, 2H, J = 10 Hz); 4.00 (AB, 2H, = 10 Hz); 6.00 (s, $-O-CH_2-CH_2-O-$);

b) <u>erfindungsgemäße Herstellung von Hydrochinon-monoäthylenglykoläther</u>

20 g des nach a) hergestellten p-Benzochinon-monoäthylenketals werden in 200 ml Methanol gelöst und unter Rühren und Eiskühlung tropfenweise mit einer Lösung von 5 g $NaBH_4$ in 100 ml Methanol so versetzt, daß die Innentemperatur 15°C nicht übersteigt. Nach beendeter Zugabe rührt man noch 30 Minuten und säuert vorsichtig mit konzentrierter HCl auf pH 4 an. Man engt die Lösung zur Trockne ein, nimmt den

Rückstand in 300 ml Äthylacetat auf und filtriert vom anfallenden NaCl ab. Die Lösung wird auf ca. 100 ml eingeengt und mit 100 ml Diäthyläther versetzt. Nach einiger Zeit setzt Kristallisation ein. Ausbeute: 18,8 g (93 %) farblose Kristalle, Schmp. 88°C).

$C_8H_{10}O_3$ (154.1) ber.: C 62.4 H 6.5
gef.: C 62.4 H 6.4

IR (KBr): 3500-2500, 1500, 1250, 1100, 1080, 1050, 980 $cm^{-1}$

$^1$H-NMR (DMSO, 60 MHz): $\tau$ = 3.3 (s, Phenyl-H), 6.2 (m, $OCH_2CH_2$-OH)

Beispiel 2

a) Herstellung des Ausgangsprodukts (4,4-Dimethoxy-cyclohexadienon)

100 g 3,3,6,6-Tetramethoxy-1,4-cyclohexadien werden in 1 l Phosphatpuffer (pH: 6.0) 30 Minuten kräftig gerührt. Man schüttelt mit 400 ml Methylenchlorid aus, trocknet über $MgSO_4$ und das Lösungsmittel ab. Ausbeute 73 g (94 %) hellgelbes Öl.

IR (NaCl): 2990, 2870, 1690, 1640, 1120, 1050 $cm^{-1}$

$^1$H-NMR ($CDCl_3$, 60 MHz): $\tau$ = 3.20 ($\underline{A}$B, 2H, J = 10 Hz), 3.70 (A$\underline{B}$, 2H, J = 10 Hz), 6.60 (s, $CH_3O$-)

b) erfindungsgemäße Herstellung des Hydrochinon-monomethyläthers

20 g des nach a) hergestellten 4,4-Dimethoxy-cyclohexadienons werden in 200 ml Methanol gelöst und unter Rühren und Eiskühlung tropfenweise mit einer Lösung von 5 g $NaBH_4$ in 100 ml Methanol so versetzt, daß die Innentemperatur 15°C nicht übersteigt. Nach beendeter Zugabe rührt man noch 30 Minuten und säuert vorsichtig mit konzentrierter HCl auf pH 4 an.

Man engt die Lösung zur Trockne ein, nimmt den Rückstand in 360 ml Äthylacetat auf und filtriert vom anfallenden NaCl ab. Die Lösung wird zur Trockne eingeengt und der Rückstand im Ölpumpenvakuum destilliert. Ausbeute 15 g (94 %).

Beispiel 3

a) Herstellung des Ausgangsprodukts (p-Benzochinon-monophthalylketal)

20 g des in Beispiel 2a) hergestellten 4,4-Dimethoxy-cyclohexadienons und 20 g Phthalylalkohol werden in 100 ml Äther aufgenommen und unter Rühren mit 2 Tropfen 70 %iger Perchlorsäure versetzt. Nach 20 Minuten Rühren bei Raumtemperatur filtriert man von den anfallenden Kristallen ab und wäscht mit wenig Äther nach. Ausbeute: 24 g (84 %) farblose Kristalle Schmp. 170°C.

| | | | |
|---|---|---|---|
| $C_{14}H_{12}O_3$ (228.2) | ber.: | C 73.7 | H 5.3 |
| | gef.: | C 73.4 | H 5.2 |

IR (KBr): 3020, 2970, 1680, 1640, 1450, 1390, 1320, 1210, 1190, 1110, 1040, 960, 860, 750 $cm^{-1}$

$^1$H-NMR ($CDCl_3$, 60 MHz): $\tau$ = 2.8 (m, Phenyl), 2.9 (AB, 4(5)H), 3,7 (AB, 2(6)H), 5.0 (s, $CH_2$-O-)

b) erfindungsgemäße Herstellung des Hydrochinon-monophthalyläthers

20 g des nach a) hergestellten Phthalylketals werden in 100 ml Methanol aufgenommen und unter Rühren und Eiskühlung tropfenweise mit einer Lösung von 4 g $NaBH_4$ in 100 ml Methanol so versetzt, daß die Innentemperatur 15°C nicht übersteigt. Nach beendeter Zugabe rührt man noch 30 Minuten und säuert vorsichtig mit konzentrierter HCl auf pH 4 an. Man engt die Lösung zur Trockne ein, nimmt in 400 ml Äthylacetat auf

und schüttelt mit 200 ml Wasser aus. Nach Trocknen über $MgSO_4$ zieht man das Lösungsmittel ab. Aus Chloroform erhält man farblose Kristalle vom Schmp. 127°C. Ausbeute 17,7 g (88 %)

$C_{14}H_{14}O_3$ (230.2) ber.: C 73.0 H 6.1
gef.: C 73.2 H 5.9

IR (KBr): 3500-2500, 1610, 1380, 1130, 1040, 1010, 820, 740 $cm^{-1}$

$^1H$-NMR (DMSO, 60 MHz): $\tau$ = 2.7 (m, Phenyl), 3.25 (m, Phenyl), 4.6 (m, OH), 5.1 (s, $CH_2$-O), 5.4 (s, $CH_2$-O)

Beispiel 4

a) Herstellung des Ausgangsprodukts (Monomethoxy-p-benzochinon-dimethylmonoketal)

50 g 1,3,3,6,6-Pentamethoxycyclohexadien-1,4 werden in einem Gemisch von 200 ml Aceton und 100 ml 2 % Essigsäure 45 Minuten bei 20°C gerührt. Man verdünnt mit 300 ml Wasser und extrahiert mit 300 ml Methylenchlorid. Nach Trocknen der organischen Phase über $MgSO_4$ zieht man das Lösungsmittel ab. Ausbeute: 31 g /78 %) hellgelbes Öl.

$C_9H_{12}O_4$ (189.1) ber.: C 58.7 H 6.5
gef.: C 58.8 H 6.4

IR (NaCl): 2980, 1670, 1640, 1600, 1130 $cm^{-1}$

$^1H$-NMR ($CDCl_3$, 60 MHz): $\tau$= 3.4 (AB), 3.7 (AB), 4.4 (m), 6.2 (s, $OCH_3$), 6.75 (s, $OCH_3$)

b) erfindungsgemäße Herstellung des Methoxyhydrochinon-monomethyläthers

30 g des nach a) anfallenden Monoketals werden in 200 ml Methanol gelöst und mit frisch bereitetem Raney-Nickel (3 g Legierung) mit $H_2$ unter Normaldruck bei 20°C hydriert. Nach Aufnahme der theoretischen Menge $H_2$ wird die Reaktion abgebrochen. Man filtriert vom Katalysator ab und engt die Lösung ein. Man erhält ein ca. 9:1-Gemisch von Hydrochinonmonoäther A und Keton B:

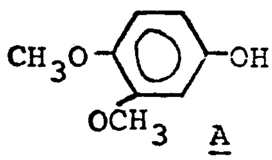 A

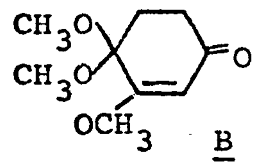 B

Gesamtausbeute: 21 g (84 %).

Beispiel 5

a) Herstellung des Ausgangsprodukts (Monochlor-p-benzochinondimethylmonoketal)

50 g 1-Chlor-3,3,6,6-Tetramethoxycyclohexadien werden in einem Gemisch von 200 ml Aceton und 100 ml 2 %ige Essigsäure 4 Stunden bei 20°C gerührt. Danach verdünnt man mit 300 ml Wasser und extrahiert mit 300 ml Methylenchlorid. Man trocknet über $MgSO_4$ und zieht das Lösungsmittel ab. Ausbeute: 39 g (98 %).

| | | | |
|---|---|---|---|
| $C_8H_9O_3Cl$ (188.5) | ber.: | C 50.9 | H 4.7 |
| | gef.: | C 50.8 | H 4.7 |

IR (NaCl): 2980, 1650, 1580, 1270, 1080, 970 $cm^{-1}$

$^1$H-NMR ($CDCl_3$, 60 MHz): $\tau$ = 3.1 - 3.8 (m, Phenyl), 6.7 (s, $OCH_3$).

b) erfindungsgemäße Herstellung des Monochlor-hydrochinonmonomethyläthers

30 g des nach a) dargestellten Monoketals werden in 200 ml Wasser aufgenommen und unter Rühren tropfenweise mit einer wässrigen Lösung von 6 g $NaBH_4$ in 100 ml Wasser versetzt. Nach

beendeter Zugabe säuert man die Reaktionslösung vorsichtig unter Eiskühlung mit konzentrierter HCl auf pH 5 an und extrahiert die Lösung 3mal mit je 300 ml Äthylacetat. Nach Trocknen der vereinigten organischen Phasen über $MgSO_4$ zieht man das Lösungsmittel ab. Ausbeute: 24,5 g (98 %).

IR (NaCl): 3550-3200 (OH), 2980, 1500, 1420, 1280, 1200, 1050, 1020, 920 $cm^{-1}$

$^1$H-NMR ($CDCl_3$, 60 MHz): $\tau$ = 3.1 - 3.4 (m, Phenyl), 4.3 - 4.6 (m, OH), 6.2 (s, $OCH_3$)

Beispiel 6

a) Herstellung des Ausgangsprodukts (p-Benzochinon-di-propandiol-1,3-ketal)

50 g Tetramethoxycyclohexadien und 55 g Propandiol-1,3 werden mit 300 ml Methylenchlorid versetzt und unter Rühren bei 20°C mit 100 mg p-Toluol-sulfonsäure versetzt. Nach 20 Minuten Reaktionszeit versetzt man mit 50 ml gesättigter Bicarbonatlösung, trennt die organische Phase ab und trocknet über $MgSO_4$. Das Lösungsmittel wird abgezogen und der Rückstand aus Diäthyläther umkristallisiert. Ausbeute: 53 g (94 %) farblose Kristalle, Schmp. 191°C.

| | | | |
|---|---|---|---|
| $C_{12}H_{16}O_4$ (224.2) | ber.: | C 64.3 | H 7.2 |
| | gef.: | C 64.4 | H 7.2 |

IR (KBr): 2990, 2980, 1420, 1230, 1120, 1070, 1040, 980, 960, 930 $cm^{-1}$

$^1$H-NMR (60 MHz, $CDCl_3$): $\tau$= 3.7 (s, 2(3,5,6)-H), 6.0 (t) und 8.2 (m) $OCH_2$-CH-CH-O).

b) Monoentketalisierung

50 g des nach a) dargestellten Diketals werden in 200 ml Aceton gelöst und mit 2 Tropfen 70 %iger Perchlorsäure versetzt. Nach 5 Minuten bricht man die Reaktion durch Zugabe von 200 ml Bicarbonatlösung ab und schüttelt mit 300 ml Methylenchlorid aus. Nach Trocknen über $MgSO_4$ zieht man das Lösungsmittel ab. Aus Äther/Petroläther umkristallisiert erhält man 30 g (82 %) farblose Kristalle.

| | | | | |
|---|---|---|---|---|
| $C_9H_{10}O_3$ | (166.0) | ber.: | C 65.0 | H 6.0 |
| | | gef.: | C 65.1 | H 5.8 |

IR (KBr): 2980, 2970, 1660, 1620, 1380, 1280, 1180, 980 $cm^{-1}$

$^1H$-NMR (60 MHz, $CDCl_3$): $\tau$ = 2.9 (AB, 2H), 3.8 (AB, 2H), 6.0 (A, 4H), 8.1 (m, 2H).

c) erfindungsgemäße Herstellung von Hydrochinon-monopropylenglykoläther

20 g des nach b) hergestellten Monoketals werden in 200 ml Methanol aufgenommen und wie in Beispiel 3b) mit 4.5 g $NaBH_4$ reduziert. Ausbeute: 18 g (91 %) farblose Kristalle, Schmp. 81°C.

| | | | | |
|---|---|---|---|---|
| $C_9H_{12}O_3$ | (168.0) | ber.: | C 64.3 | H 7.1 |
| | | gef.: | C 64.1 | H 6.9 |

IR (KBr): 3600-3100 (OH), 1520, 1420, 1240, 1090, 1050, 980 $cm^{-1}$

$^1H$-NMR (60 MHz, $CDCl_3$): $\tau$ = 3.4 (s, Phenyl), 6.15 (A, 2H), 6.55 (A, 2H), 8.2 (m, 2H).

Beispiel 7

a) erfindungsgemäße Herstellung des Hydrochinon-monoäthylenglykoläthers

10 g des in Beispiel 6b) hergestellten Monoketals werden in 100 ml wasserfreiem Tetrahydrofuran gelöst und unter Rühren zu einer Suspension von 1g $LiAlH_4$ in 50 ml wasserfreiem Tetrahydrofuran so zugetropft, daß die Innentemperatur nicht über 20°C steigt. Nach beendeter Zugabe rührt man noch 2 Stunden. Man bricht die Reaktion durch vorsichtige Zugabe von Wasser ab. Danach säuert man mit 2n $H_2SO_4$ auf pH 2 an und extrahiert die Lösung zweimal mit je 300 ml Äthylacetat. Nach Trocknen über $MgSO_4$ zieht man das Lösungsmittel ab. Ausbeute: 65 g (64 %) farblose Kristalle.

Beispiel 8

a) Herstellung des Ausgangsprodukt (p-Benzochinon-dimethylmonoketal)

50 g Tetramethoxycyclohexadien und 30 g $Na_2S_2O_5$ werden in 500 ml wässrigem Phosphatpuffer (pH 5,5) aufgenommen und 5 Stunden bei 20°C gerührt. Danach schüttelt man zweimal mit je 300 ml Äthylacetat aus, trocknet die vereinigten organischen Phasen über $MgSO_4$ und zieht das Lösungsmittel ab. Ausbeute: 23 g (60 %)

b) erfindungsgemäße Herstellung des Hydrochinon-monomethyl-äthers

30 g des nach a) hergestellten Dimethoxymonoketals und 20 g $Na_2S_2O_5$ werden in 300 ml Wasser aufgenommen und 5 Stunden bei 20°C gerührt. Man extrahiert zweimal mit je 300 ml Äthylacetat, trocknet die vereinigten organischen Phasen über $MgSO_4$ und zieht das Lösungsmittel ab. Ausbeute: 22,5 g (74 %).

Beispiel 9

a) Herstellung und Isolierung eines erfindungsgemäßen Chinolketals

20 g des nach Beispiel 2 a hergestellten 4,4-Dimethoxycyclohexadienons werden in 100 ml Diethylether auf aufgenommen und unter Rühren und Eiskühlung tropfenweise mit einer Lösung von 2.5 g $NaBH_4$ in 50 ml Wasser vernetzt. Nach 2 h Reaktionszeit trennt man die organische Phase ab, trocknet über $MgSO_4$ und zieht das Lösungsmittel ab. Ausbeute: 18 g (92 %) farbloses Öl.

IR (NaCl): 3600 - 3200, 1400, 1220, 1110, 1060, 960 $cm^{-1}$

$^1$H-NMR ($CDCl_3$, 60 MHz): $\tau$ = 3.80 (A̲B, 2H) 4.20 (AB̲ , 2H), 5.60 (m, 1H), 6.60 (m, OH), 6.75 (s, $OCH_3$), 6.80 (s, $OCH_3$)

b) erfindungsgemäße Herstellung des Hydrochinonmonomethylethers

15 g des nach a) hergestellten Chinolketals werden in 50 ml Methanol aufgenommen und mit einem Tropfen konz. HCl vernetzt. Man zieht das Lösungsmittel ab. Ausbeute: 12 g (100%) hellgelbes Öl.

1. Verfahren zur Herstellung von Hydrochinonmonoäthern, dadurch gekennzeichnet, daß man
   a) p-Benzochinon-monoketale in einem inerten Lösungsmittel zu den entsprechenden Chinolketalen reduziert und
   b) diese - gegebenenfalls nach ihrer Isolierung - dann mit einer Säure behandelt.

2. Verfahren nach Anspruch 1, dadurch gekennzeichnet, daß man als p-Benzochinonmonoketale Verbindungen der Formel I

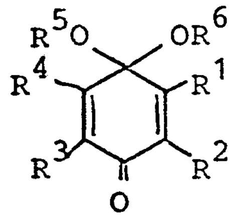

(I)

worin die Reste $R^1$ bis $R^4$ unabhängig veoneinander Wasserstoff, Halogen, oder-gegebenenfalls über Sauerstoff an den Chinonring gebundene-organische Reste bedeuten, sowie $R^5$ und $R^6$ - ebenfalls unabhängig voneinander - gegebenenfalls durch reaktionsinerte Gruppen substituierte Alkylreste oder - zusammen - einen gegebenenfalls durch O-Atome unterbrochenen Alkylenrest oder einen Arylenrest bedeuten, verwendet.

3. Verfahren nach den Ansprüchen 1 bis 2, dadurch gekennzeichnet, daß man als p-Benzochinonmonoketale Verbindungen der Formel I verwendet, worin mindestens 3 der Reste $R^1$ bis $R^4$
   = H und - wenn nur 3 dieser Reste = H - ist der 4.
   = Cl, Br, $(C_1-C_4)$-Alkyl oder $(C_1-C_4)$-Alkoxy, insbesondere ist der 4. Rest nur = Cl, Br oder $(C_1-C_4)$-Alkyl.

4. Verfahren nach den Ansprüchen 1 bis 3, dadurch gekennzeichnet, daß man als p-Benzochinonmonoketale Verbindungen

der Formel I verwendet, worin $R^5 = R^6 = (C_1\text{-}C_4)$-Alkyl sowie $R^5 + R^6$ = Alkylengruppen mit 2 - 4 C-Atomen in der Kette oder 1,2-Phenylen bedeuten.

5. Verfahren nach den Ansprüchen 1 bis 4, dadurch gekennzeichnet, daß man als inerte Lösungsmittel Wasser, $(C_1\text{-}C_3)$-Alkanole und/oder aliphatische $(C_3\text{-}C_8)$-Äther verwendet.

6. Verfahren nach den Ansprüchen 1 bis 5, dadurch gekennzeichnet, daß man die Reduktion mit einem komplexen Hydrid des Bors oder Aluminiums, mit einem Sulfit oder mit molekularem Wasserstoff in Gegenwart eines Nickel-Katalysators bis zur Aufnahme von einem Mol Wasserstoff/Mol p-Benzochinonmonoketal durchführt.

7. Verfahren nach den Ansprüchen 1 bis 6, dadurch gekennzeichnet, daß man die Reduktion bei Temperaturen zwischen etwa -10 und +25°C, vorzugsweise zwischen etwa +5 und +15°C, durchführt.

8. Verfahren nach den Ansprüchen 1 bis 7, dadurch gekennzeichnet, daß man für die Säurebehandlung in Stufe b) Salzsäure verwendet.

9. Chinolketale der Formel II

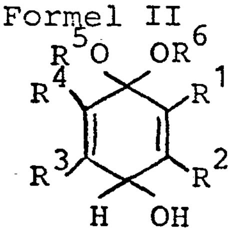

(II),

worin die Reste $R^1$ bis $R^6$ die gleiche Bedeutung wie in Formel I besitzen.

10. Chinolketale der Formel II nach Anspruch 9, wobei in der Formel mindestens 3 der Reste $R^1$ bis $R^4$ = H und - wenn nur 3 dieser Reste = H sind - ist der 4.

= Cl, Br, ($C_1$-$C_4$)-Alkyl oder ($C_1$-$C_4$)-Alkoxy, insbesondere ist der 4. Rest nur = Cl, Br oder ($C_1$-$C_4$)-Alkyl und

$R^5$ = $R^6$ = ($C_1$-$C_4$)-Alkyl sowie $R^5$ + $R^6$ = Alkylengruppen mit 2 - 4 C-Atomen in der Kette oder 1,2-Phenylen bedeuten

Europäisches Patentamt

# EUROPÄISCHER RECHERCHENBERICHT

0039484

Nummer der Anmeldung

EP 81103259.8

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | DE - A1 - 2 547 464 (HOECHST)<br>+ Patentansprüche 1-3, 5-7; Seite 6, letzter Absatz +<br>-- | 1,5-7 | C 07 C 43/23<br>C 07 C 43/295<br>C 07 C 41/18<br>C 07 C 43/315 |
| | DE - A1 - 2 740 718 (EASTMAN KODAK)<br>+ Patentanspruch 1 +<br>-- | 1 | |
| | DE - A1 - 2 548 384 (UBE INDUSTRIES)<br>+ Patentanspruch 1; Beispiel 1 +<br>-- | 1 | |
| | DE - C - 827 803 (BASF)<br>+ Patentanspruch; Beispiel 4 +<br>-- | 1 | |
| | CH - A - 534 649 (ICI)<br>+ Patentanspruch +<br>-- | 1 | |
| | US - A - 3 846 501 (JEROME A. VESELY)<br>+ Patentanspruch 1 +<br>---- | 1 | |

RECHERCHIERTE SACHGEBIETE (Int. Cl.³)

C 07 C 41/00
C 07 C 43/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 03-07-1981 | REIF |